# DEMANDE DE BREVET EUROPEEN

(11) **EP 1 757 264 A2**
(43) Date de publication de la demande: **28.02.2007**
(21) Numéro de dépôt: 06291334.8
(22) Date de dépôt: 22.08.2006
(51) Int. Cl.: A61K 8/49, A61Q 5/10, C09B 29/00

(54) **Colorants azoïques cationiques à motifs julolidine composition tinctoriale les contenant procédé de coloration**

(30) Priorité: 23.08.2005 FR 0508695
(71) Demandeur: L'Oreal, 75008 Paris (FR)
(72) Inventeur: Daubresse, Nicolas, 78170 La celle Saint Cloud (FR); Greaves, Andrew, 77144 Montevrain (FR)
(74) Mandataire: Casalonga, Axel

(57) **Abrégé**

La présente demande concerne l'utilisation pour la coloration des fibres kératiniques, en particulier des fibres kératiniques humaines, telles que les cheveux de composés azoïques cationiques à motifs julolidine à titre de colorants directs,

La présente demande concerne également une composition tinctoriale pour la teinture des fibres kératiniques, en particulier des fibres kératiniques humaines telles que les cheveux comprenant dans un milieu de teinture approprié au moins un colorant direct azoïques cationiques à motifs julolidine.

La présente demande concerne aussi un procédé de teinture des fibres kératiniques, en particulier des fibres kératiniques humaines telles que les cheveux mettant en oeuvre la composition tinctoriale selon l'invention.

La présente demande concerne aussi certains composés azoïques cationiques particuliers à motifs julolidine.

## Description

La présente demande a pour objet l'utilisation pour la coloration des fibres kératiniques, en particulier des fibres kératiniques humaines, telles que les cheveux de composés azoïques cationiques à motifs julolidine à titre de colorants directs, une composition tinctoriale contenant ces composés azoïques particuliers, un procédé de coloration desdites fibres les mettant en oeuvre, un dispositif à compartiment et de nouveaux composés azoïques cationiques à motifs julolidine.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs, tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser pauser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes sont mises en oeuvre sans la présence obligatoire d'un agent oxydant. Ces teintures peuvent être effectuées de manière répétée sans dégrader la fibre kératinique.

Il est connu par exemple d'utiliser des colorants directs nitrés benzèniques, anthraquinoniques, nitropyridiniques, azoïques, xanthéniques, acridiniques, aziniques ou triarylméthaniques.

Il en résulte des colorations souvent chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une mauvaise tenue aux lavages ou à la transpiration.

Il existe un réel besoin de disposer de colorants plus performants permettant des améliorations en terme d'homogénéité de teinture, en fonction de la qualité des cheveux colorés, de résistance aux shampooings et après-shampooing (ténacité), de limitation de dégorgement qui induit des risques de tâches, de virage de couleur dans le temps quand on associe ensemble des colorants chromatiques de ténacités respectives différentes.

De plus, l'utilisation de colorants directs cationiques connus permet l'obtention de nuances chromatiques puissantes, mais limitées à un champ coloriel (jaune, orange, rouge). Il existe donc un besoin de colorants situés en dehors du champ coloriel connu.

De manière surprenante et avantageuse, la Demanderesse vient de découvrir qu'une classe de colorants directs azoïques cationiques à motif dit « julolidine » permet ces améliorations.

Des colorants cationiques à motif dit « julolidine » sont décrits dans le brevet US 4,341,853 en tant que photosensibilisateur en électrophotographie.

Ces composés azoïques conduisent à des teintures résistantes aux agents extérieurs (soleil, intempéries) ainsi qu'aux shampooings et à la transpiration. Ces compositions présentent un bon profil toxicologique. En outre, ces colorants permettent d'obtenir des reflets intenses, notamment dans les coloris bleus et violets. Seuls ou en combinaison avec d'autres colorants traditionnels, directs ou d'oxydation, ils permettent d'étendre les gammes de couleurs.

Un premier objet de la présente invention porte sur l'utilisation des composés azoïques cationiques à motif julolidine à titre de colorants directs pour les fibres kératiniques, en particulier les fibres kératiniques humaines, telles que les cheveux.

Un second objet de la présente invention consiste en une composition tinctoriale pour la teinture des fibres kératiniques, en particulier des fibres kératiniques humaines, telles que les cheveux comprenant dans un milieu de teinture approprié pour la teinture au moins un composé azoïque cationique à motif julolidine selon l'invention.

L'invention porte également sur un procédé de teinture des fibres kératiniques, en particulier des fibres kératiniques humaines, telles que les cheveux mettant en oeuvre la composition tinctoriale selon l'invention.

Un autre objet de la présente invention est l'utilisation de ladite composition pour la teinture des fibres kératiniques, en particulier des fibres kératiniques humaines, telles que les cheveux.

L'invention concerne de plus un dispositif à compartiment ou « kit » comprenant la composition tinctoriale selon la présente invention.

Enfin, un dernier objet de l'invention concerne des composés cationiques azoïques à motif julolidine particuliers.

D'autres caractéristiques, aspects, objets et avantages de l'invention apparaîtront encore plus clairement à la lecture de la description et des exemples qui suivent.

Il est à noter que dans ce qui va suivre, et à moins d'une autre indication, les bornes d'un domaine de valeurs sont comprises dans ce domaine.

Les composés azoïques cationiques à motif julolidine selon la présente invention, correspondent au composé de formule (I) suivante : 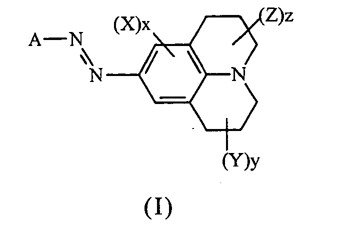 dans laquelle
A représente un hétérocycle aromatique cationique choisi parmi les composés de formules générales suivantes : 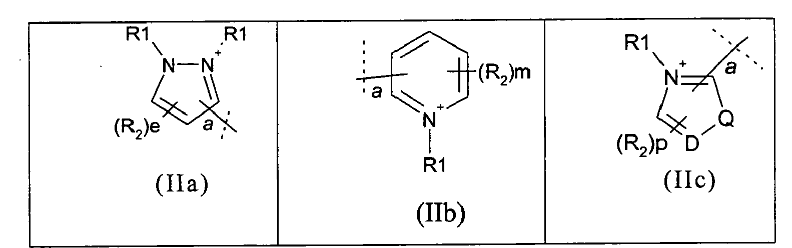 R₁ représente indépendamment les uns des autres:
- une chaîne hydrocarbonée en C₁-C₁₆, linéaire ou ramifiée, saturée ou insaturée, pouvant former un ou plusieurs cycles carbonés comportant de 3 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de liaison nitro, nitroso, peroxyde et diazo ; R₁ étant directement rattaché à l'atome d'azote, quaternisé ou non, du cycle hétéroaromatique A par l'intermédiaire d'un atome de carbone. R₂ représente indépendamment les uns des autres:
- une chaîne hydrocarbonée en C₁-C₁₆, linéaire ou ramifiée, saturée ou insaturée, pouvant former un ou plusieurs cycles carbonés aromatique ou non, comportant de 3 à 6 chaînons, éventuellement substituée, éventuellement interrompue par un ou plusieurs hétéroatomes ou par un ou plusieurs groupements portant au moins un hétéroatome, de préférence choisis parmi l'oxygène, l'azote ;
- un groupement hydroxyle,
- un groupement alkyl-oxy en C₁-C₄,
- un groupement (poly)hydroxyalkyloxy en C₂-C₄ ;
- un groupement alkyloxycarbonyle (R₁₁O-CO-) dans lequel R₁₁ représente un radical alkyle en C₁-C₄ ;
- un radical alkylcarbonyloxy (R₁₂CO-O-) dans lequel R₁₂ représente un radical alkyle est en C₁-C₄;
- un groupement amino, un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
- un groupement alkylcarbonylamino (R₁₃CO-NR₁₃-) et/ou (R₁₃CO-NH) dans lequel les radicaux R₁₃ indépendamment l'un de l'autre, représentent un radical alkyle en C₁-C₄;
- un groupement carbamoyle ((R₁₄)₂N-CO) dans lequel les radicaux R₁₄ indépendamment l'un de l'autre , identiques ou non, représentent un atome d'hydrogène, un radical alkyle en C₁-C₄;
- un groupement uréido ((R₁₅)₂N-CO-NR₁₆-) dans lequel les radicaux R₁₅ et R₁₆, indépendamment l'un de l'autre, représentent un atome d'hydrogène, un radical alkyle en C₁-C₄;
- un groupement sulfonamide ((R₁₇)₂N-SO₂-) dans lequel les radicaux R₁₇, indépendamment l'un de l'autre, représentent un atome d'hydrogène, un radical alkyle en C₁-C₄, ;
- un groupement alkylsulfonylamino (R₁₈SO₂-NR₁₉-) dans lequel les radicaux R₁₈, R₁₉, indépendamment l'un de l'autre, représentent un atome d'hydrogène, un radical alkyle en C₁-C₄;
- un groupement guanidinium ((R₂₀)₂N-C(=NH₂+)-NR₂₁-) dans lequel les radicaux R₂₀ et R₂₁, indépendamment les uns des autres, représentent un atome d'hydrogène, un radical alkyle en C₁-C₄;
- un groupement nitro ;
- un groupement cyano ;
- un atome d'halogène, de préférence le chlore, le fluor ;
deux radicaux R₂, portés par des atomes de carbone adjacents peuvent former ensemble avec l'atome de carbone auquel chacun est rattaché, un cycle condensé, aromatique ou non ;
m représente un entier compris entre 0 et 4 ;
e est un entier compris entre 0 et 2 ;
p est un entier entre 0 et 1 ;
D représente un groupe CR₂ ou un atome d'azote
Q représente un groupe NR₁, ou un atome d'oxygène ou de soufre ;
la liaison a issue des formules (IIa), (IIb) ou (IIc), relie le groupement A au groupement azoïque ;
dans le cas des formules (IIa), (IIb) ou (IIc) et lorsque deux radicaux R₂ portés par deux atomes de carbone adjacents forment un cycle aromatique, la liaison a peut relier le groupement A au groupement azoïque par l'intermédiaire dudit cycle aromatique ;
l'électroneutralité des composés étant assurée par un ou plusieurs anions An⁻, identiques ou non, cosmétiquement acceptables, parmi lesquels le chlorure, méthylsulfate, méthosulfate, tosylate, acétate ;
X, Y et Z sont définis comme possibilités de substitutions respectivement sur les cycles alkyles et aryle du noyau tricyclique ;
x est compris entre 0 et 2, y est compris entre 0 et 6, et z est compris entre 0 et 6, x, y et z étant entiers ;
ou leurs sels d'addition ou leurs solvates,
à l'exception de
2-(9-julolidylazo)-3-méthylbenzothiazolium perchlorate
2-(9-julolidylazo)-3-éthylthiazolium perchlorate, connus comme sensibilisateurs en électrophotographie.
Dans ce qui va suivre, et à moins d'une indication différente :
- Lorsqu'un radical alkyle ou la partie alkyle d'un radical est dit(e) 'substitué(e)', alors il ou elle comprend au moins un substituant choisi parmi les groupements :
   - hydroxyle,
   - alkyloxy en C₁-C₄, (poly)hydroxyalkyloxy en C₂-C₄,
   - amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.
- Lorsqu'un radical aryle ou hétéroaryle ou la partie aryle ou hétéroaryle d'un radical est dit(e) 'substitué(e)', comme par exemple le substituant X sur le noyau aromatique du tricycle, alors il ou elle comprend au moins un substituant porté par un atome de carbone, choisi parmi :
   • un radical alkyle en C₁-C₁₆, de préférence en C₁-C₈, éventuellement substitué par un ou plusieurs radicaux choisis parmi les radicaux hydroxy, alkyloxy en C₁-C₂, (poly)-hydroxyalkyloxy en C₂-C₄, acylamino, amino substitué par un ou deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l' azote ;
   • un atome d'halogène tel que chlore, fluor ou brome ;
   • un groupement hydroxyle ;
   • un radical alkyloxy en C₁-C₂ ;
   • un radical (poly)-hydroxyalkyloxy en C₂-C₄ ;
   • un radical amino ;
   • un radical amino substitué par un ou deux radicaux alkyles, identiques ou différents, en C₁-C₄ éventuellement porteurs d'au moins un groupement hydroxyle;
   • un radical acylamino (-N₃₁R-COR₃₂) dans lequel le radical R₃₁ est un atome d'hydrogène, un radical alkyle en C₁-C₄ éventuellement porteur d'au moins un groupement hydroxyle et le radical R₃₂ est un radical alkyle en C₁-C₂ ;
   • un radical carbamoyle ((R₃₃)₂N-CO-) dans lequel les radicaux R₃₃, identiques ou non, représentent un atome d'hydrogène, un radical alkyle en C₁-C₄ éventuellement porteur d'au moins un groupement hydroxyle ;
   • un radical alkylsulfonylamino (R₃₄SO₂-N₃₅R-) dans lequel le radical R₃₄ représente un atome d'hydrogène, un radical alkyle en C₁-C₄ éventuellement porteur d'au moins un groupement hydroxyle et le radical R₃₅ représente un radical alkyle en C₁-C₄, un radical phényle ;
   • un radical aminosulfonyle ((R₃₆)₂N-SO₂-) dans lequel les radicaux R₃₆, identiques ou non, représentent un atome d'hydrogène, un radical alkyle en C₁-C₄ éventuellement porteur d'au moins un groupement hydroxyle.
- Lorsque la partie cyclique ou hétérocyclique d'un radical non aromatique est dit(e) 'substitué(e)', comme par exemple les substituants Y et Z des hétérocycles aliphatiques du tricycle, alors il ou elle comprend au moins un substituant porté par un atome de carbone choisi parmi les groupements :
   - hydroxyle,
   - alkyloxy en C₁-C₄, (poly)hydroxyalkyloxy en C₂-C₄,
   - alkylcarbonylamino ((R₄₁CO-NR₄₂-) dans lequel le radical R₄₂ est un atome d'hydrogène, un radical alkyle en C₁-C₄ éventuellement porteur d'au moins un groupement hydroxyle et le radical R₄₁ est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.
- Lorsqu'un cycle ne porte pas le nombre maximum de substituants, alors la ou les positions non susbtituées portent un atome d'hydrogène.

De préférence, R₁ représente un groupement alkyle ou hydroxyalkyle en C₁-C₈.

Selon une première variante, les formules (IIa), (IIb) et (IIc) sont telles qu'elles comportent deux radicaux R₂ portés par des atomes de carbone adjacents, ces dits radicaux formant alors ensemble avec l'atome de carbone auquel chacun est rattaché, un cycle condensé aromatique, éventuellement substitué.

Selon une deuxième variante, e, m et p valent 0.

De préférence, x vaut 0 ou 1 avec X un groupe alkyle, hydroxyle, hydroxyalkyle, alkyloxy, amino, alkylamino, dialkylamino, acylamino ; alkyle signifiant une chaîne en C₁-C₆ éventuellement substituée, acyle signifiant alkylcarbonyle.

A titre d'exemple de composés de formule (I) utilisables selon l'invention, on peut citer les composés suivants :

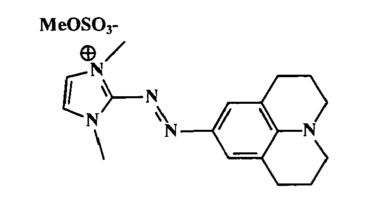

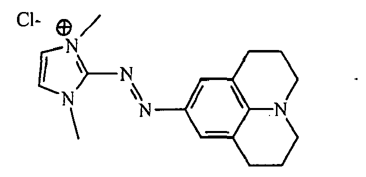

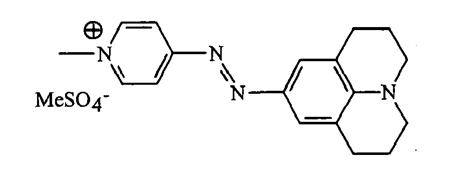

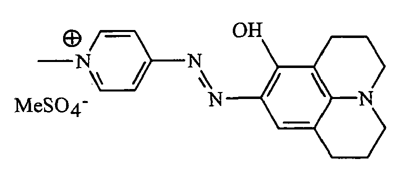

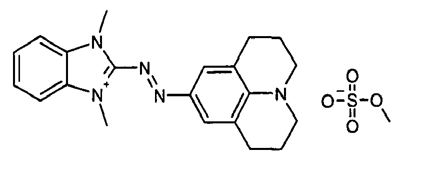

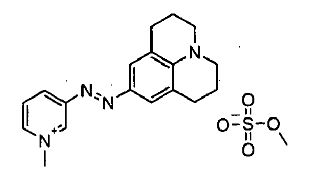

Les colorants répondant à la formule peuvent être obtenus, par exemple, par quatre voies de synthèse différentes, par la suite appelées respectivement voie A, voie B, voie C et voie D:

Dans les schémas ci-après, la définition de B, précurseur non cationique de A, répond aux 3 formules suivantes: 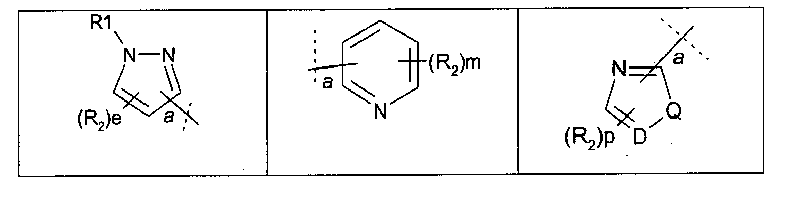 Dans ce qui suit, HAL signifie un groupement halogène relié à R₁ par l'un de ses atomes de carbone, de préférence un groupement chloro, bromo ou iodo, ou bien un groupement alkylsulfato, de préférence méthylsulfato ou éthylsulfato ou bien méthylsulfonato (mésylate) ou arylsulfonato (tosylate)

### 1/Voie de synthèse A

Le composé de formule (I) est obtenu en trois étapes successives conformément au schéma ci après :

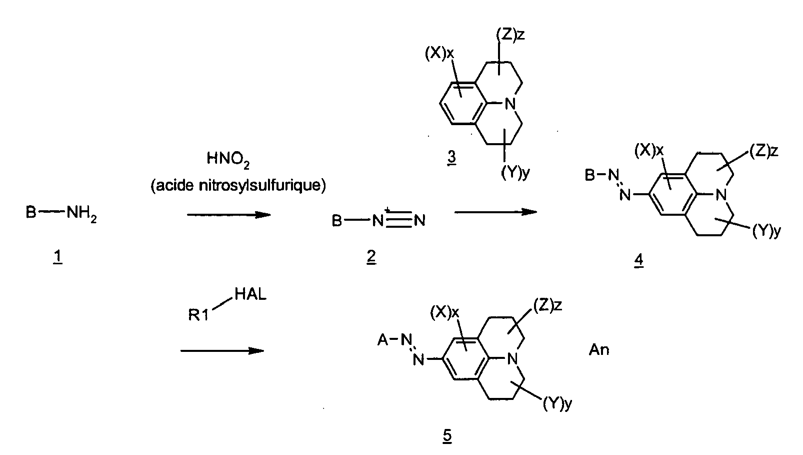

On prépare le sel de diazonium 2 de l'amine hétéroaromatique BNH₂ 1 par les méthodes classiques (H Zollinger, Color Chemistry, Wiley VCH Ed 2003 et The Chemistry of synthetic dyes, Academic Press, London, vol II, 1952). On fait ensuite réagir le sel de diazonium 2 sur un composé 3 (un amine aromatique à motif julolidine) pour former le composé 4. Ce type de couplage est bien connu dans la littérature citée ci-dessus.

La troisième étape consiste à faire réagir le composé 4 avec un réactif d'alkylation, tel qu'un sulfate d'alkyle, un halogénure d'alkyle, un alkylsulfonate d'alkyle ou un arylsulfonate d'alkyle, pour former le composé 5 : un colorant direct azoïque cationique à motif julolidine .

La réaction d'alkylation s'effectue par exemple dans un solvant halogéné (dichlorométhane) ou ester (acétate d'éthyle), à température inférieure à 150°C, de préférence à reflux du solvant. Ces conditions sont notamment décrites dans la littérature. En référence à ce type de réaction, on peut citer par exemple Advanced Organic Synthesis 5th Ed M. Smith and J. March John Wiley & Sons Ed, 2001 et la demande internationale WO 03/060015.

### 2/Voie de synthèse B

Le composé de formule (I) peut être obtenu par diazotation du dérivé para-aminé d'une amine aromatique à motif julolidine 6, puis couplage du sel de diazonium 7 obtenu avec un hétérocyle pour conduire au composé 8, puis alkylation de la fonction hétérocyclique pour conduire au composé 9 (le colorant direct azoïque cationique à motif julolidine) selon le schéma suivant :

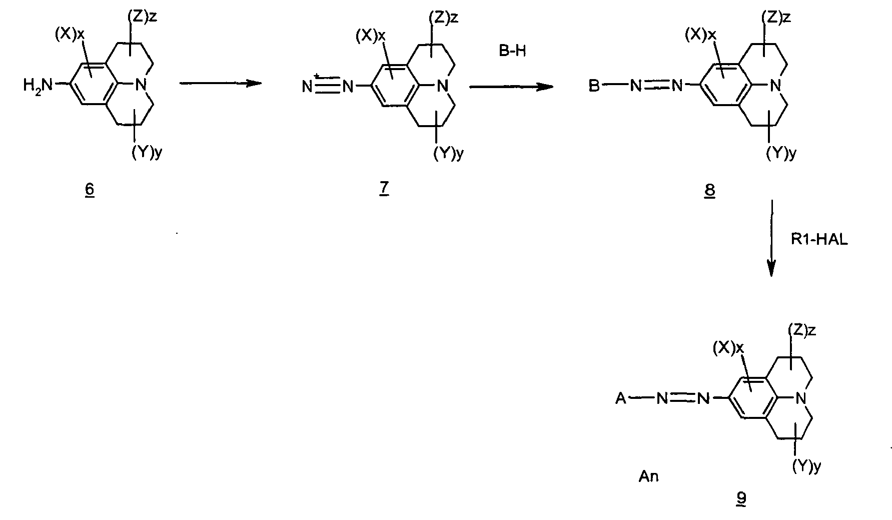

Toutes les étapes de synthèse sont décrites dans la littérature et les références de la voie 1 sont aussi applicables.

Plus particulièrement, la deuxième étape consiste à faire réagir le sel de diazonium 7 préalablement obtenu avec un hétérocycle B-H (suivant la définition précédente de B et H représentant un atome d'hydrogène lié à B sur la position qui sera ensuite celle du groupement azo).

### 3/Voie de synthèse C

Le composé de formule (I) peut également être obtenu dans deux étapes de synthèse : attaque nucléophile sur un atome de carbone d'un hétérocycle cationique [11] par un composé para-hydrazino d'une amine aromatique à motif julolidine 10 puis oxydation du composé 12 obtenu pour conduire au composé 13 (un colorant direct azoïque cationique à motif julolidine). Ces réactions sont exemplifiées ci-dessous par un hétérocycle cationique de type pyridinium et plus spécifiquement avec le composé 11 selon le schéma suivant :

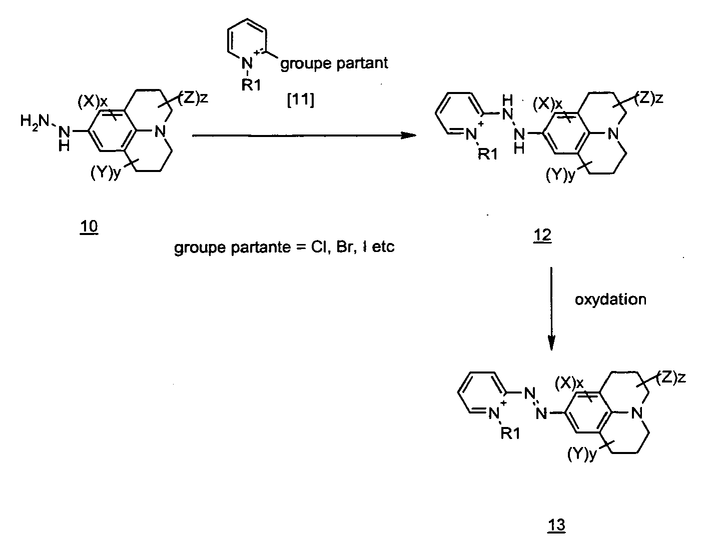

La réaction pour former le composé 12 peut s'effectuer dans un solvant polaire, de préférence un alcool ou la DMF, à une température inférieure à 150°C, en présence le cas échant d'une base moins nucléophile que l'hydrazine choisie (J. Med. Chem. 1996, 39 (2), 570-581). L'oxydation peut s'effectuer à l'aide des oxydants usuels : N-bromosuccinimide, chlorure ferrique, oxyde de manganèse, oxydes de chrome, eau oxygénée, peracides, de préférence en milieu acide et à température inférieure à 100°C pendant moins de vingt-quatre heures.

### 4/Voie de synthèse D

Le composé de formule (I) peut également être obtenu par couplage oxydatif. Cette voie est décrite dans la littérature. En référence à ce type de réaction, on peut citer par exemple Angew. Chem. 1958, 70,215 ; Angew. Chem., Int. Ed., 1962,1,640 et H Zollinger, Color Chemistry 3èmè Ed, VCH Wiley, 2003. La voie est exemplifiée selon le schéma suivant utilisant pour illustrer l'hétérocycle pyridinium, et plus spécifiquement le composé 14 pour conduire au composé 17 (un colorant direct azoïque cationique à motif julolidine):

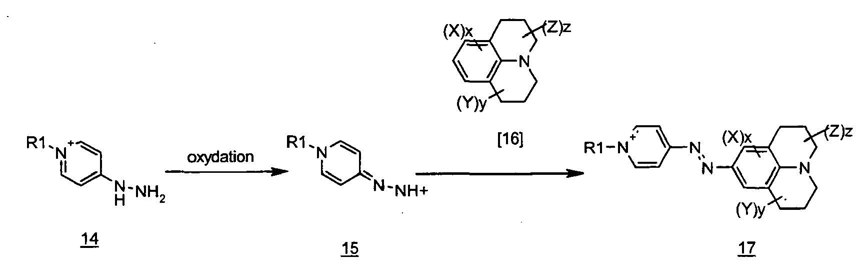

Les réactifs de départs sont commerciaux ou sont obtenus suivant les méthodes connues de l'homme de l'art, avantageusement à partir de composés disponibles dans le commerce.

L'invention porte également sur l'utilisation de composés azoïques cationiques à motif julolidine correspondant au composé de formule (I) suivante : 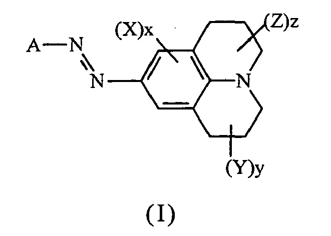 dans laquelle
A représente un hétérocycle aromatique cationique choisi parmi les composés de formules générales suivantes : 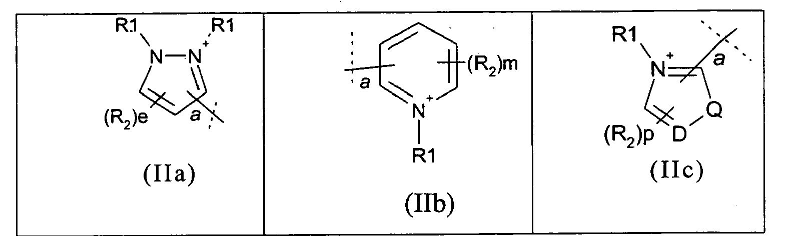 R₁ représente indépendamment les uns des autres:
- une chaîne hydrocarbonée en C₁-C₁₆, linéaire ou ramifiée, saturée ou insaturée, pouvant former un ou plusieurs cycles carbonés comportant de 3 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de liaison nitro, nitroso, peroxyde et diazo ; R₁ étant directement rattaché à l'atome d'azote, quaternisé ou non, du cycle hétéroaromatique A par l'intermédiaire d'un atome de carbone.
   R₂ représente indépendamment les uns des autres:
- une chaîne hydrocarbonée en C₁-C₁₆, linéaire ou ramifiée, saturée ou insaturée, pouvant former un ou plusieurs cycles carbonés aromatique ou non, comportant de 3 à 6 chaînons, éventuellement substituée, éventuellement interrompue par un ou plusieurs hétéroatomes ou par un ou plusieurs groupements portant au moins un hétéroatome, de préférence choisis parmi l'oxygène, l'azote ;
- un groupement hydroxyle,
- un groupement alkyl-oxy en C₁-C₄,
- un groupement (poly)hydroxyalkyloxy en C₂-C₄ ;
- un groupement alkyloxycarbonyle (R₁₁O-CO-) dans lequel R₁₁ représente un radical alkyle en C₁-C₄ ;
- un radical alkylcarbonyloxy (R₁₂CO-O-) dans lequel R₁₂ représente un radical alkyle est en C₁-C₄;
- un groupement amino, un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
- un groupement alkylcarbonylamino (R₁₃CO-NR₁₃-) et/ou (R₁₃CO-NH) dans lequel les radicaux R₁₃ indépendamment l'un de l'autre, représentent un radical alkyle en C₁-C₄;
- un groupement carbamoyle ((R₁₄)₂N-CO) dans lequel les radicaux R₁₄ indépendamment l'un de l'autre , identiques ou non, représentent un atome d'hydrogène, un radical alkyle en C₁-C₄;
- un groupement uréido ((R₁₅)₂N-CO-NR₁₆-) dans lequel les radicaux R₁₅ et R₁₆, indépendamment l'un de l'autre, représentent un atome d'hydrogène, un radical alkyle en C₁-C₄;
- un groupement sulfonamide ((R₁₇)₂N-SO₂-) dans lequel les radicaux R₁₇, indépendamment l'un de l'autre, représentent un atome d'hydrogène, un radical alkyle en C₁-C₄, ;
- un groupement alkylsulfonylamino (R₁₈SO₂-NR₁₉-) dans lequel les radicaux R₁₈, R₁₉, indépendamment l'un de l'autre, représentent un atome d'hydrogène, un radical alkyle en C₁-C₄;
- un groupement guanidinium ((R₂₀)₂N-C(=NH₂+)-NR₂₁-) dans lequel les radicaux R₂₀ et R₂₁, indépendamment les uns des autres, représentent un atome d'hydrogène, un radical alkyle en C₁-C₄;
- un groupement nitro ;
- un groupement cyano ;
- un atome d'halogène, de préférence le chlore, le fluor ;
deux radicaux R₂, portés par des atomes de carbone adjacents peuvent former ensemble avec l'atome de carbone auquel chacun est rattaché, un cycle condensé, aromatique ou non ;
m représente un entier compris entre 0 et 4 ;
e est un entier compris entre 0 et 2 ;
p est un entier entre 0 et 1 ;
D représente un groupe CR₂ ou un atome d'azote
Q représente un groupe NR₁, ou un atome d'oxygène ou de soufre ;
la liaison a issue des formules (IIa), (IIb) ou (IIc), relie le groupement A au groupement azoïque ;
dans le cas des formules (IIa), (IIb) ou (IIc) et lorsque deux radicaux R₂ portés par deux atomes de carbone adjacents forment un cycle aromatique, la liaison a peut relier le groupement A au groupement azoïque par l'intermédiaire dudit cycle aromatique ;
l'électroneutralité des composés étant assurée par un ou plusieurs anions An⁻, identiques ou non, cosmétiquement acceptables, parmi lesquels le chlorure, méthylsulfate, méthosulfate, tosylate, acétate ;
X, Y et Z sont définis comme possibilités de substitutions respectivement sur les cycles alkyles et aryle du noyau tricyclique ;
x est compris entre 0 et 2, y est compris entre 0 et 6, et z est compris entre 0 et 6, x, y et z étant entiers ;
ou leurs sels d'addition ou leurs solvates
Dans ce qui va suivre, et à moins d'une indication différente :
- Lorsqu'un radical alkyle ou la partie alkyle d'un radical est dit(e) 'substitué(e)', alors il ou elle comprend au moins un substituant choisi parmi les groupements :
   - hydroxyle,
   - alkyloxy en C₁-C₄, (poly)hydroxyalkyloxy en C₂-C₄,
   - amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.
- Lorsqu'un radical aryle ou hétéroaryle ou la partie aryle ou hétéroaryle d'un radical est dit(e) 'substitué(e)', comme par exemple le substituant X sur le noyau aromatique du tricycle, alors il ou elle comprend au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alkyloxy en C₁-C₂, (poly)-hydroxyalkyloxy en C₂-C₄, acylamino, amino substitué par un ou deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
   - un atome d'halogène tel que chlore, fluor ou brome ;
   - un groupement hydroxyle ;
   - un radical alkyloxy en C₁-C₂ ;
   - un radical (poly)-hydroxyalkyloxy en C₂-C₄ ;
   - un radical amino ;
   - un radical amino substitué par un ou deux radicaux alkyles, identiques ou différents, en C₁-C₄ éventuellement porteurs d'au moins un groupement hydroxyle;
   - un radical acylamino (-N₃₁R-COR₃₂) dans lequel le radical R₃₁ est un atome d'hydrogène, un radical alkyle en C₁-C₄ éventuellement porteur d'au moins un groupement hydroxyle et le radical R₃₂ est un radical alkyle en C₁-C₂ ;
   - un radical carbamoyle ((R₃₃)₂N-CO-) dans lequel les radicaux R₃₃, identiques ou non, représentent un atome d'hydrogène, un radical alkyle en C₁-C₄ éventuellement porteur d'au moins un groupement hydroxyle ;
   - un radical alkylsulfonylamino (R₃₄SO₂-N₃₅R-) dans lequel le radical R₃₄ représente un atome d'hydrogène, un radical alkyle en C₁-C₄ éventuellement porteur d'au moins un groupement hydroxyle et le radical R₃₅ représente un radical alkyle en C₁-C₄, un radical phényle ;
   - un radical aminosulfonyle ((R₃₆)₂N-SO₂-) dans lequel les radicaux R₃₆, identiques ou non, représentent un atome d'hydrogène, un radical alkyle en C₁-C₄ éventuellement porteur d'au moins un groupement hydroxyle.
- Lorsque la partie cyclique ou hétérocyclique d'un radical non aromatique est dit(e) 'substitué(e)', comme par exemple les substituants Y et Z des hétérocycles aliphatiques du tricycle, alors il ou elle comprend au moins un substituant porté par un atome de carbone choisi parmi les groupements :
   - hydroxyle,
   - alkyloxy en C₁-C₄, (poly)hydroxyalkyloxy en C₂-C₄,
   - alkylcarbonylamino ((R₄₁CO-NR₄₂-) dans lequel le radical R₄₂ est un atome d'hydrogène, un radical alkyle en C₁-C₄ éventuellement porteur d'au moins un groupement hydroxyle et le radical R₄₁ est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.
- Lorsqu'un cycle ne porte pas le nombre maximum de substituants, alors la ou les positions non susbtituées portent un atome d'hydrogène.

De préférence, R₁ représente un groupement alkyle ou hydroxyalkyle en C₁-C₈.

Selon une première variante, les formules (IIa), (IIb) et (IIc) sont telles qu'elles comportent deux radicaux R₂ portés par des atomes de carbone adjacents, ces dits radicaux formant alors ensemble avec l'atome de carbone auquel chacun est rattaché, un cycle condensé aromatique, éventuellement substitué.

Selon une deuxième variante, e, m et p valent 0.

De préférence, x vaut 0 ou 1 avec X un groupe alkyle, hydroxyle, hydroxyalkyle, alkyloxy, amino, alkylamino, dialkylamino, acylamino ; alkyle signifiant une chaîne en C₁-C₆ éventuellement substituée, acyle signifiant alkylcarbonyle.

A titre d'exemple de composés de formule (I) utilisables selon l'invention, on peut citer les composés suivants :

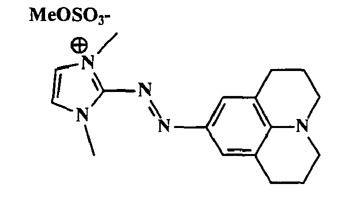

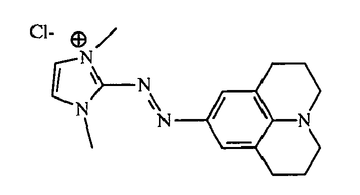

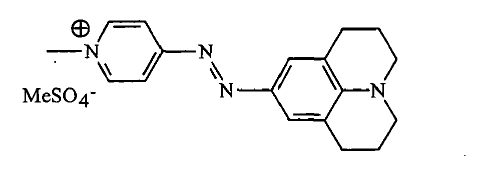

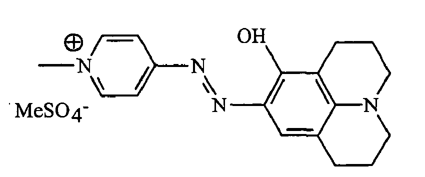

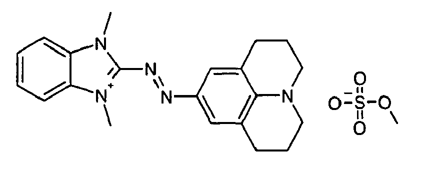

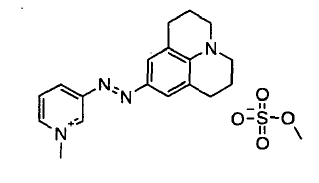

L'invention porte également sur une composition tinctoriale pour la teinture des fibres kératiniques, en particulier des fibres kératiniques humaines, telles que les cheveux comprenant dans un milieu de teinture approprié, au moins un composé azoïque cationique tel que défini ci-dessus pour l'utilisation en tant que colorant direct.

La composition tinctoriale selon la présente invention peut comprendre de 0,001 à 20 %, de préférence de 0,01 à 10 % en poids de colorant direct azoïque cationique de formule (I) par rapport au poids total de la composition.

La composition de la présente invention peut comprendre en outre au moins une base d'oxydation.

A titre d'exemple, les bases d'oxydation sont choisies parmi les phénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxypyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

Si la composition contient au moins une base d'oxydation, la composition selon l'invention contient de préférence un ou plusieurs coupleurs conventionnellement utilisés pour la teinture de fibres kératiniques. Parmi ces coupleurs, on peut notamment citer les méta-phénylènediamines, les méta-aminphénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs additionnels autres que les colorants directs azoïques cationiques de formule (I) selon l'invention, pouvant notamment être choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels. A titre d'exemples non limitatifs, on peut citer les colorants benzéniques nitrés, les colorants azoïques, azométhiniques, méthiniques, tétraazapentaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Parmi les colorants directs benzéniques utilisables selon l'invention, on peut citer de manière non limitative les composés suivants :
- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

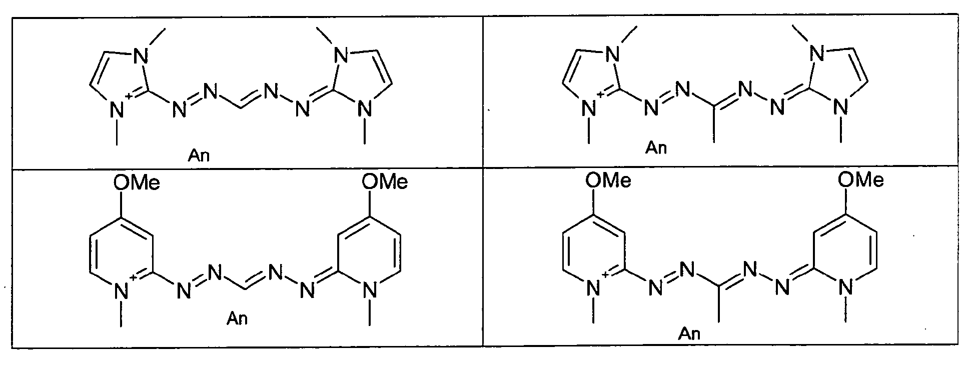

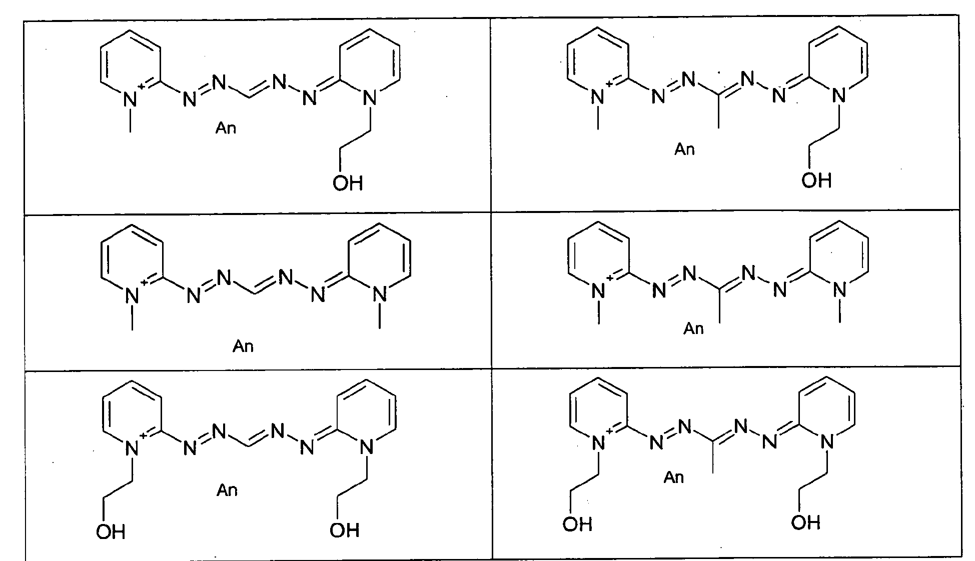

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs différents de ceux de formule (I) représentent de préférence de 0,001 à 20 % en poids environ du poids total de la composition, et encore plus préférentiellement de 0,005 à 10 % en poids environ.

La composition selon l'invention peut également comprendre au moins un agent oxydant classiquement utilisé pour la teinture d'oxydation des fibres kératiniques, comme par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, le monométhyléther de propylèneglycol, le monoéthyléther et le monométhyléther du diéthylènegl.ycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs et des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12, de préférence entre 5 et 11, et encore plus particulièrement de 6 à 10,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines, telles que les mono-, di- et triéthanolamines ainsi que leurs dérivés, les hydroxydes de sodium ou de potassium et les composés de formule (II) suivante : 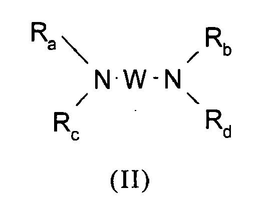 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, puis on rince lesdites fibres.

Selon un mode de réalisation particulier, la composition de l'invention est appliquée sur les fibres kératiniques en présence d'un agent oxydant, on parle alors dans ce cas d'un procédé de coloration éclaircissante. L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

De préférence, dans ce mode de réalisation particulier, la composition de l'invention contient au moins une base d'oxydation.

Les compositions avec ou sans oxydant sont appliquées sur les matières kératiniques et après un temps de pause de 3 minutes à 1 heure environ, de préférence 15 minutes à 45 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, encore plus préférentiellement entre 5 et 11 et encore plus particulièrement entre 6 et 10,5. Il peut être ajusté à la valeur désirée au moyen d'agents acidifiants ou alcalinisants habituellement utilisés en coloration des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une coloration des fibres kératiniques, et notamment des cheveux humains.

L'invention porte également sur l'utilisation de la composition tinctoriale selon l'invention pour la teinture des fibres kératiniques.

L'invention a aussi pour objet un dispositif à plusieurs compartiments ou "kit" de coloration, dans lequel un premier compartiment renferme la composition tinctoriale selon l'invention et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

Les exemples suivants servent à illustrer l'invention sans toutefois présenter un caractère limitatif.

### Exemples de préparations de composés selon l'invention :

### 1) Exemple 1 par la voie A

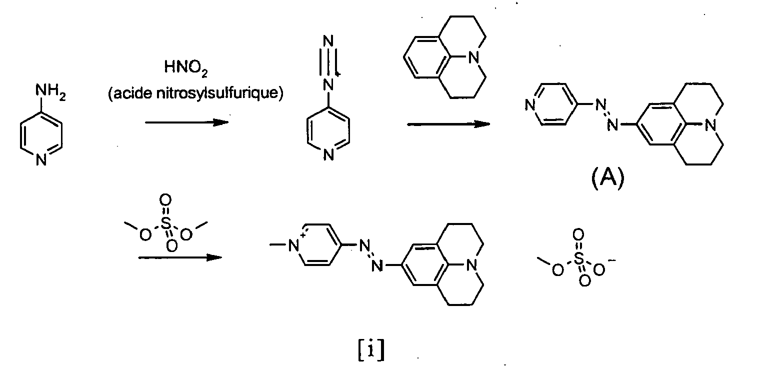

A 200 g de solution à 40% p/v d'acide nitrosylsulfurique dans l'acide sulfurique, on ajoute 100 mL d'acide acétique anhydre et 500 mL d'acide propionique anhydre, puis on amène le mélange à une température inférieure à 5°C, on introduit progressivement 50 g de 4-aminopyridine. Le mélange ainsi obtenu est dilué par addition de 200 mL d'acide propionique et 40 mL d'acide acétique, puis on ajoute goutte à goutte, en maintenant la température inférieure à 10°C, une solution de 50 g de julolidine dans 200 mL de méthanol. Le mélange réactionnel est neutralisé après 16h de réaction à une température inférieure à 5°C par introduction de 500 mL d'eau, 300 g de glace puis 500 mL de soude à 30%. Le produit (A) est extrait à l'acétate d'éthyle, purifié par chromatographie sur gel de silice.

A 5 g de produit (A) pur, dilués dans 50 mL de dichlorométhane, on ajoute 5 mL de diméthylsulfate, puis on maintient le mélange réactionnel sous agitation pendant 2 h. Le milieu est concentré par distillation sous vide des solvants, puis le résidu obtenu est purifié par solubilisation dans la méthyléthylcétone suivie de précipitation à l'heptane. On obtient ainsi 7.12 g d'une poudre bleue. Les analyses l'indiquent conforme à l'attendu [i].

### 2) Exemple 2 par la voie A

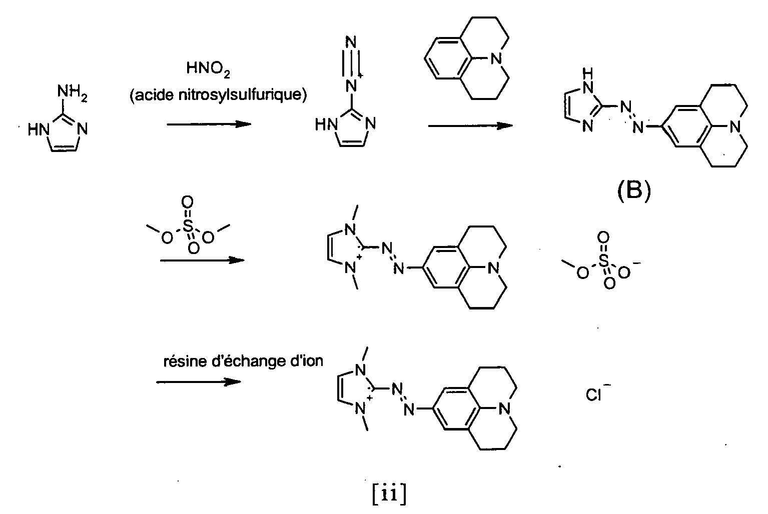

A 13.21 g de suspension d'aminoimidazole dans 100 mL d'eau, on ajoute 30 mL d'acide chlorhydrique à 35% on refroidit le mélange à - 5°C puis on ajoute goutte à goutte une solution de 7 g de nitrite de sodium dans 10 mL d'eau, en maintenant une température inférieure à 0°C. On ajoute alors 5 g d'acide sulfanilique puis on verse le mélange sur une solution acide de julolidine obtenue au préalable par mélange de 8.75 g de julolidine, 175 mL d'eau et 225 mL d'acide acétique et 40 g d'acétate de sodium et maintenue à 0°C. Après 2 h à 5°C, le mélange réactionnel est dilué, ramené à pH 8 par de la soude. Le produit (B), précipité, est filtré et lavé à l'eau.

5 g de (B) sont solubilisés dans 50 mL de dichlorométhane ; sont successivement ajoutés : 5 mL de diméthylsulfate et 1.53 g d'acétate de sodium. Après 2 h d'agitation, le mélange est concentré sous vide, repris dans 100 mL de méthyléthylcétone puis on ajoute 100 mL d'heptane. Le précipité obtenu est lavé par de l'heptane, on recueille 4.5 g de poudre violet noir. On procède ensuite à un échange d'ions sur résine pour obtenir le produit attendu [ii] sous forme de chlorhydrate (2.56 g). Les analyses l'indiquent conforme à l'attendu [ii].

### 3) Exemple 3 par la voie A

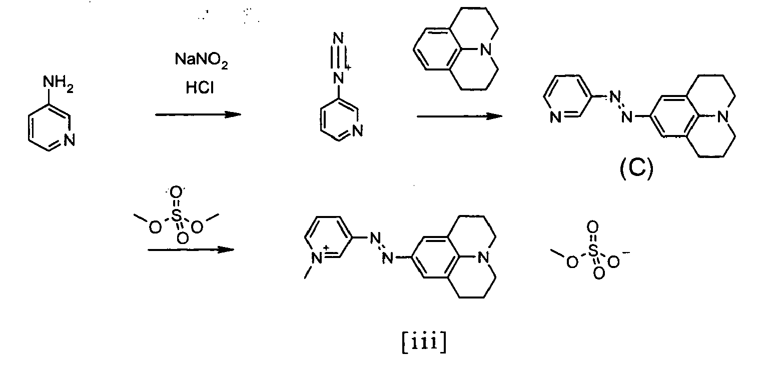

A 6.3 g de 3-aminopyridine, 17 mL d'acide chlorhydrique à 35% et 65 g de glace sont mélangés dans un tricol de 500 mL. Une solution de 5.08 g de nitrite de sodium dans 20 mL d'eau est ajoutée goutte à goutte, en maintenant la température du mélange à 0°C. Une fois l'addition achevée, le mélange est maintenu agité 20 min à 0°C puis une solution de 400 mg d'urée dans 10 mL d'eau est ajoutée. Une solution de julolidine 11.6 g dans un mélange éthanol (25 mL) acide chlorhydrique 35% (16 mL), glace (22 g) est ajoutée goutte à goutte en conservant une température inférieure à 10°C. Après 3 h à 5°C, le mélange réactionnel est neutralisé par ajout d'ammoniaque, jusqu'à obtention d'un pH de 9.5. La pâte ainsi obtenue est extraite par de l'acétate d'éthyle avec addition d'eau. La phase organique est séchée et concentrée sous vide ; Le produit ainsi obtenu est purifié par chromatographie (gel de silice, eluant heptane/acétate d'ethyle) et on recueille une fraction pure d'une poudre de couleur rouge foncée (4.3 g), conforme à l'attendu (produit (C)).

Le produit obtenu lors de l'étape ci-dessus (127 mg) est solubilisé dans le toluène (5 mL), 86 µL de diméthylsulfate sont ajoutés et le mélange réactionnel est maintenu agité pendant 72 h. Le toluène est éliminé sous vide, le produit obtenu est traité par de l'eau pendant 4 h à température ambiante puis séché sous vide, on obtient un solide violet foncé (170 mg). Les analyses l'indiquent conforme à l'attendu [iii].

### 4) Exemple 4 par la voie A

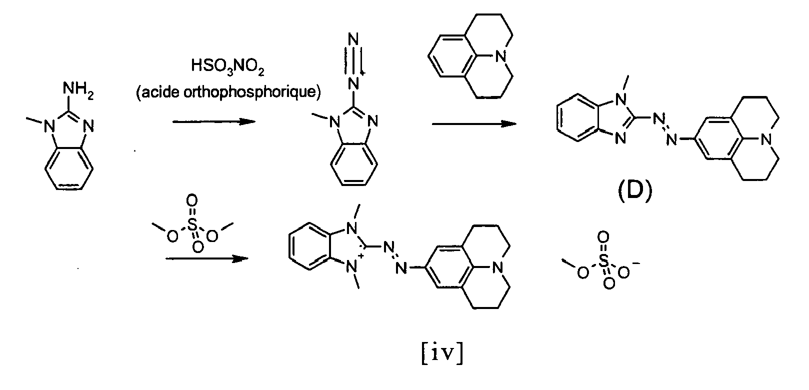

10 g de 2-amino-1-methyl benzimidazole sont solubilisés dans un mélange de 60ml d'acide ortho phosphorique et 20 ml d'acide acétique à chaud.

26 g d'acide nitrosyle sulfurique (à 40% dans l'acide sulfurique) sont dilués dans 25 mL d'acide phosphorique 98% à 10°C dans un tricol de 250 mL muni d'un thermomètre et d'une arrivée d'argon.

La première solution est versée goutte à goutte sur la seconde, en maintenant la température entre 2 et 5°C en 30 min. Le mélange est agité pendant 15min. 3.3 g d'acide sulfamique sont ajoutés et le mélange est agité pendant 15 min.

13 g de julolidine préalablement fondue sont solubilisés dans 25 mL de DMF et 9 g d'acide acétique.

Le sel de diazonium obtenu lors de l'opération précédente est versé goutte à goutte sur cette solution en maintenant une température inférieure à 10°C. Le mélange réactionnel est dilué avec 50 mL d'eau puis maintenu à 0°C pendant 18 h. 500 g de glace sont ajoutés puis 100 mL de soude à 30%; à nouveau 500 g de glace et 150 mL de soude à 30%. Le mélange ainsi obtenu est encore dilué par 2 L d'eau puis ramené à pH 7 par addition d'hydrogénocarbonate de sodium (190 g).

Le précipité obtenu est filtré (9.6 g) et séché. 1 g est purifié par chromatographie (silice, éluant dichlorométhane/méthanol). On obtient 100 mg d'une poudre rouge. Les analyses l'indiquent conforme à l'attendu (D).

100 mg de D sont solubilisés dans 20 mL de dichlorométhane, et on ajoute à ce mélange, goutte à goutte, 500 µl de diméthyl sulfate à température ambiante. La solution est agitée pendant 5 min. puis versée sur 250 mL d'éther éthylique. Le précipité est filtré et lavé avec 4 x 50 mL d'éther éthylique. Après séchage, on récupère 50 mg d'une pâte noire. Les analyses l'indiquent conforme à l'attendu [iv].

### 5) Exemple 5 par la voie A

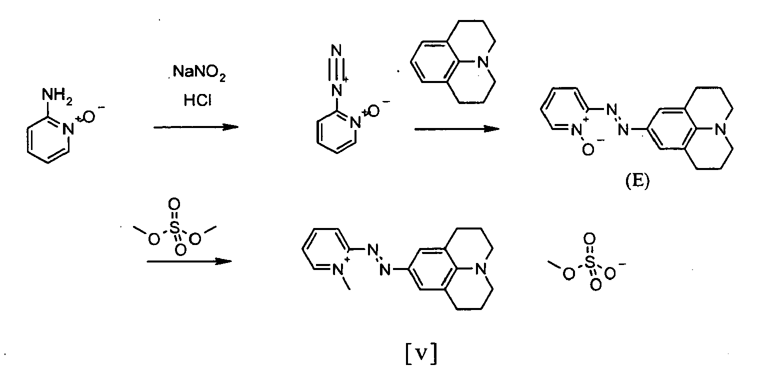

11,8 g de julolidine sont dissous à 40°C dans 100 mL d'eau additionnée de 5,7 mL d'acide chlorhydrique à 35%. 10 g de pyridin-2-amine 1-oxide (préparés suivant les méthodes décrites dans Synth. Commun. 1977, 509-514) sont dissous dans 100 mL d'eau additionnée de 17,4 mL d'acide chlorhydrique à 35%. Une solution de 4,7 g de nitrite de sodium dissous dans 10 mL d'eau est ajouté, en maintenant la température du mélange à 0°C. Le mélange est maintenu agité 30 min à 0°C. Il est ensuite versé sur la solution acide de julolidine, en maintenant la température inférieure à 10°C. Le mélange est ramené à température ambiante en deux heures. L'addition de 70 g d'acétate de sodium permet de ramener le pH du mélange à 4,2. Après extraction au dichlorométhane, rinçage à l'eau, séchage sur sulfate de sodium, filtration, concentration sous vide, trituration dans l'éther, filtration de la poudre obtenue et séchage sous vide, 10 g de poudre violet foncé sont recueillis. Les analyses indiquent qu'elle est conforme au produit (E) attendu, avec des impuretés minoritaires.

5 g de (E) sont dissous dans 20 mL de N-méthylpyrrolidinone (NMP) et la solution est chauffée à 60°C. 4,05 g de diméthylsulfate sont ajoutés au mélange. Après 2 h 30 min. la solution est refroidie à température ambiante. 50 mL de solution ammoniacale à 20% sont ajoutés. Après 15 h à température ambiante, le mélange est extrait au dichlorométhane (2 x 100 mL), lavé à l'eau, séché et concentré sous vide. L'huile bleu foncé obtenue est lavée deux fois à l'éther isopropylique, puis triturée dans une troisième fraction. Après filtration, rinçage à l'éther et séchage sous vide 2 g de poudre brune sont recueillis. Les analyses indiquent qu'elle est conforme au produit [v] attendu.

### Exemples de teintures (conditions non éclaircissantes):

On a préparé les compositions tinctoriales dans les proportions suivantes :
Solution 1

| | |
|---|---|
| Hydroxyéthylcellulose Natrosol 250MR | 0.72 g |
| Alkyl C8/C10(50 :50) hydroxyethylcellulose CG110 | 5 g |
| Alcool benzylique | 4 g |
| Polyethylène glycol 400 | 4 g |
| Eau | Qsp 100 g |

Solution 2 : TAMPON pH 9,5

| | |
|---|---|
| Chlorure d'ammonium (NH₄Cl) | 5,4 g |
| Ammoniaque en sol. à 20% | qsp pH 9,5 (env. 4 mL) |
| Eau déminéralisée | qsp 100 mL |

Solution 3 : TAMPON pH 7

| | |
|---|---|
| KH₂PO₄ | 0,026 mol/L |
| Na₂PO₄ | 0,041 mol/L |
| Eau déminéralisée | qsp 500 mL |

Les compositions de coloration sont obtenues en dissolvant le colorant indiqué ci dessous (5x10⁻³mol/L) dans la solution 1 puis en ajoutant un volume équivalent de solution tampon 2 ou 3 (pH 7 ou 9,5).

Chaque composition est appliquée sur des cheveux gris à 90% de blancs, (1 g de mèche pour 6 g de solution). Après 30 min de pause, les mèches sont rincées, lavées avec un shampooing standard, rincées à nouveau puis séchées.

Les résultats de teinture suivants ont été obtenus :

| | pH 7 | pH 9,5 |
|---|---|---|
| Colorant [i] | Bleu chromatique intense | Bleu chromatique intense |
| Colorant [ii] | Violet chromatique intense | Violet chromatique intense |
| Colorant [iii] | Fuchsia chromatique intense | Fuchsia chromatique intense |
| Colorant [iv] | Bleu chromatique intense | Bleu chromatique intense |
| Colorant [v] | Bleu-violet intense | Bleu-violet intense |

### Exemples de teintures (conditions éclaircissantes):

On a préparé les compositions tinctoriales dans les proportions suivantes :

| | |
|---|---|
| Colorant | 0.25 g |
| Hydroxyéthylcellulose Natrosol 250MR | 0.72 g |
| Alkyl C8/C10(50 :50) hydroxyethylcellulose | 5 g |
| CG110 | |
| Alcool benzylique | 4 g |
| Polyethylène glycol 400 | 4 g |
| Hydroxyde d'Ammonium | 13 g |
| Eau | Qsp 100 g |

Au moment de l'emploi, cette formulation est mélangée poids pour poids à de l'eau oxygénée 40 volumes puis appliquée sur mèches BN (Blanc Naturel) et BP (Blanc Permanenté) - 1 g de mèche pour 6 g de solution. Après 30 min de pause, les mèches sont rincées, lavées avec un shampooing standard, rincées à nouveau puis séchées.

Les résultats de teinture suivants ont été obtenus

| | BN | BP |
|---|---|---|
| Colorant [i] | Bleu chromatique intense | Bleu chromatique intense |
| Colorant [ii] | Violet chromatique intense | Violet chromatique intense |

Les mèches ainsi colorées sont soumises à un test de résistance aux lavages qui consiste à effectuer 12 shampooings (avec un shampooing standard) et à évaluer la couleur après ces 12 shampooings. Après 12 shampooings, les mèches sont toujours colorées.

## Revendications

1. Utilisation d'au moins un composé cationique azoïque à motif julolidine de formule (I) : 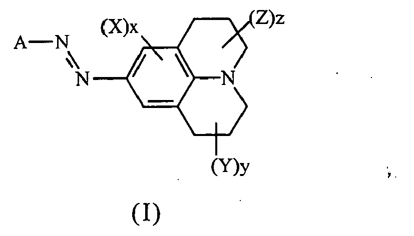 dans laquelle
A représente un hétérocycle aromatique cationique choisi parmi les composés de formules générales suivantes : 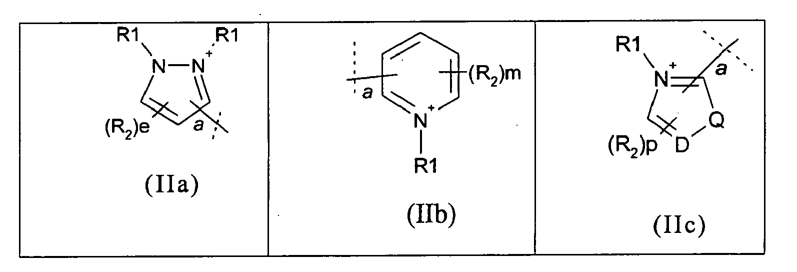 dans lesquelles :
R₁ représentent indépendamment les uns des autres :
• une chaîne hydrocarbonée en C₁-C₁₆, linéaire ou ramifiée, saturée ou insaturée, pouvant former un ou plusieurs cycles carbonés comportant de 3 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de liaison nitro, nitroso, peroxyde et diazo ; R₁ étant directement rattaché à l'atome d'azote, quaternisé ou non, du cycle hétéroaromatique A par l'intermédiaire d'un atome de carbone.
R₂ représente indépendamment les uns des autres :
• une chaîne hydrocarbonée en C₁-C₁₆, linéaire ou ramifiée, saturée ou insaturée, pouvant former un ou plusieurs cycles carbonés aromatique ou non, comportant de 3 à 6 chaînons, éventuellement substituée, éventuellement interrompue par un ou plusieurs hétéroatomes ou par un ou plusieurs groupements portant au moins un hétéroatome, de préférence choisis parmi l'oxygène, l'azote ;
• un groupement hydroxyle,
• un groupement alkyl-oxy en C₁-C₄,
• un groupement (poly)hydroxyalkyloxy en C₂-C₄ ;
• un groupement alkyloxycarbonyle (R₁₁O-CO-) dans lequel R₁₁ représente un radical alkyle en C₁-C₄,
• un radical alkylcarbonyloxy (R₁₂CO-O-) dans lequel R₁₂ représente un radical alkyle est en C₁-C₄;
• un groupement amino, un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
• un groupement alkylcarbonylamino (R₁₃CO-NR₁₃-) et/ou (R₁₃CO-NH-) dans lequel les radicaux R₁₃ indépendamment l'un de l'autre, représentent un radical alkyle en C₁-C₄;
• un groupement carbamoyle ((R₁₄)₂N-CO) dans lequel les radicaux R₁₄ indépendamment l'un de l'autre , identiques ou non, représentent un atome d'hydrogène, un radical alkyle en C₁-C₄;
• un groupement uréido ((R₁₅)₂N-CO-NR₁₆-) dans lequel les radicaux R₁₅ et R₁₆, indépendamment l'un de l'autre, représentent un atome d'hydrogène, un radical alkyle en C₁-C₄;
• un groupement sulfonamide ((R₁₇)₂N-SO₂-) dans lequel les radicaux R₁₇, indépendamment l'un de l'autre, représentent un atome d'hydrogène, un radical alkyle en C₁-C₄, ;
• un groupement alkylsulfonylamino (R₁₈SO₂-NR₁₉-) dans lequel les radicaux R₁₈, R₁₉, indépendamment l'un de l'autre, représentent un atome d'hydrogène, un radical alkyle en C₁-C₄;
• un groupement guanidinium ((R₂₀)₂N-C(=NH₂+)-NR₂₁-) dans lequel les radicaux R₂₀ et R₂₁, indépendamment les uns des autres, représentent un atome d'hydrogène, un radical alkyle en C₁-C₄;
• un groupement nitro ;
• un groupement cyano ;
• un atome d'halogène, de préférence le chlore, le fluor ;
deux radicaux R₂, portés par des atomes de carbone adjacents peuvent former ensemble avec l'atome de carbone auquel chacun est rattaché, un cycle condensé, aromatique ou non ;
m représente un entier compris entre 0 et 4 ;
e est un entier compris entre 0 et 2 ;
p est un entier entre 0 et 1 ;
D représente un groupe CR₂ ou un atome d' azote ;
Q représente un groupe NR₁, ou un atome d'oxygène ou de soufre ;
la liaison a issue des formules (IIa), (IIb) ou (IIc), relie le groupement A au groupement azoïque ;
dans le cas des formules (IIa), (IIb) ou (IIc) et lorsque deux radicaux R₂ portés par deux atomes de carbone adjacents forment un cycle aromatique, la liaison a peut relier le groupement A au groupement azoïque par l'intermédiaire dudit cycle aromatique ;
l'électroneutralité des composés étant assurée par un ou plusieurs anions An⁻, identiques ou non, cosmétiquement acceptables ;
X, Y et Z sont définis comme possibilités de substitutions respectivement sur les cycles alkyles et aryle du noyau tricyclique ;
x est compris entre 0 et 2, y est compris entre 0 et 6, et z est compris entre 0 et 6, x, y et z étant entiers ;
ou leurs sels d'addition ou leurs solvates,
lorsqu'un radical alkyle ou la partie alkyle d'un radical est dit(e) 'substitué(e)', alors il ou elle comprend au moins un substituant choisi parmi les groupements :
• hydroxyle,
• alkyloxy en C₁-C₄, (poly)hydroxyalkyloxy en C₂-C₄,
• amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote ;
lorsqu'un radical aryle ou hétéroaryle ou la partie aryle ou hétéroaryle d'un radical_est dit(e) 'substitué(e)', comme par exemple le substituant X sur le noyau aromatique du tricycle, alors il ou elle comprend au moins un substituant porté par un atome de carbone, choisi parmi :
• un radical alkyle en C₁-C₁₆, de préférence en C₁-C₈, éventuellement substitué par un ou plusieurs radicaux choisis parmi les radicaux hydroxy, alkyloxy en C₁-C₂, (poly)-hydroxyalkyloxy en C₂-C₄, acylamino, amino substitué par un ou deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
• un atome d'halogène tel que chlore, fluor ou brome ;
• un groupement hydroxyle ;
• un radical alkyloxy en C₁-C₂;
• un radical (poly)-hydroxyalkyloxy en C₂-C₄ ;
• un radical amino ;
• un radical amino substitué par un ou deux radicaux alkyles, identiques ou différents, en C₁-C₄ éventuellement porteurs d'au moins un groupement hydroxyle;
• un radical acylamino (-N₃₁R-COR₃₂) dans lequel le radical R₃₁ est un atome d'hydrogène, un radical alkyle en C₁-C₄ éventuellement porteur d'au moins un groupement hydroxyle et le radical R₃₂ est un radical alkyle en C₁-C₂ ;
• un radical carbamoyle ((R₃₃)₂N-CO-) dans lequel les radicaux R₃₃, identiques ou non, représentent un atome d'hydrogène, un radical alkyle en C₁-C₄ éventuellement porteur d'au moins un groupement hydroxyle ;
• un radical alkylsulfonylamino (R₃₄SO₂-N₃₅R-) dans lequel le radical R₃₄ représente un atome d'hydrogène, un radical alkyle en C₁-C₄ éventuellement porteur d'au moins un groupement hydroxyle et le radical R₃₅ représente un radical alkyle en C₁-C₄, un radical phényle ;
• un radical aminosulfonyle ((R₃₆)₂N-SO₂-) dans lequel les radicaux R₃₆, identiques ou non, représentent un atome d'hydrogène, un radical alkyle en C₁-C₄ éventuellement porteur d'au moins un groupement hydroxyle ;
lorsque la partie cyclique ou hétérocyclique d'un radical non aromatique est dit(e) 'substitué(e)', comme par exemple les substituants Y et Z des hétérocycles aliphatiques du tricycle, alors il ou elle comprend au moins un substituant porté par un atome de carbone choisi parmi les groupements :
• hydroxyle,
• alkyloxy en C₁-C₄, (poly)hydroxyalkyloxy en C₂-C₄,
• alkylcarbonylamino ((R₄₁CO-NR₄₂-) dans lequel le radical R₄₂ est un atome d'hydrogène, un radical alkyle en C₁-C₄ éventuellement porteur d'au moins un groupement hydroxyle et le radical R₄₁ est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote ;
lorsqu'un cycle ne porte pas le nombre maximum de substituants, alors la ou les positions non susbtituées portent un atome d'hydrogène ;
en tant que colorant direct pour les fibres kératiniques, en particulier les fibres kératiniques humaines, telles que les cheveux.

2. Utilisation selon la revendication 1, **caractérisé en ce que** R₁ représente un groupement alkyle ou hydroxyalkyle en C₁-C₈.

3. Utilisation selon la revendication 1 ou 2, **caractérisé en ce que** les formules (IIa), (IIb) et (IIc) sont telles qu'elles comportent deux radicaux R₂ portés par des atomes de carbone adjacents, ces dits radicaux formant alors ensemble avec l'atome de carbone auquel chacun est rattaché, un cycle condensé aromatique éventuellement substitué.

4. Utilisation selon l'une quelconque des revendications 1 à 3, **caractérisé en ce que** e, m et p valent 0.

5. Utilisation selon l'une quelconque des revendications 1 à 4, **caractérisé en ce** x vaut 0 ou x vaut 1 avec X représentant un groupe alkyle, hydroxyle, hydroxyalkyle, alkyloxy, amino, alkylamino, dialkylamino, acylamino.

6. Utilisation selon l'une quelconque des revendications 1 à 5, **caractérisé en ce que** le composé de formule (I) possède l'une des structures suivantes :

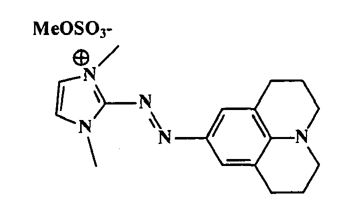

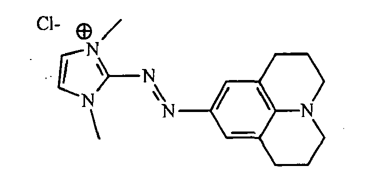

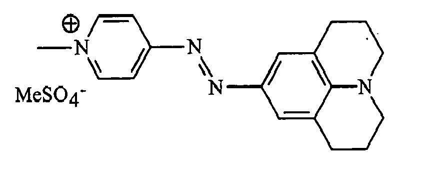

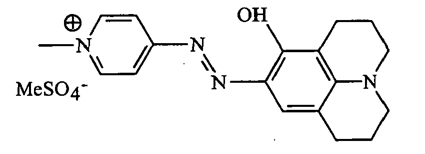

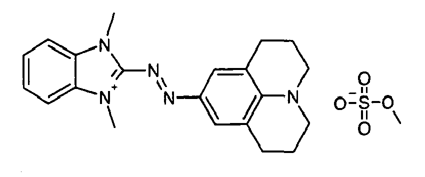

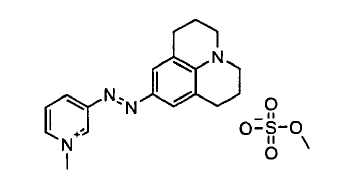

7. Composition tinctoriale pour la teinture des fibres kératiniques, en particulier des fibres kératiniques humaines telles que les cheveux comprenant, dans un milieu de teinture approprié, au moins un composé cationique azoïque tel que défini à l'une quelconque des revendications 1 à 6

8. Composition selon la revendication 7, **caractérisée en ce qu'**elle comprend de 0,001 à 20 %, de préférence de 0,01 à 10 % de colorant(s) direct(s) de formule (I) par rapport au poids total de la composition.

9. Composition selon la revendication 7 ou 8, **caractérisée en ce qu'**elle comprend une base d'oxydation choisie parmi les para-phénylènediamines, les bis-phénylalkylènediamines, les para-aminophénols, les ortho-aminophénols, les bases hétérocycliques et leurs sels d'addition.

10. Composition selon l'une quelconque des revendications 7 à 9, **caractérisée en ce qu'**elle comprend une quantité comprise entre 0,001 à 20 % en poids et de préférence entre 0,005 et 6% en poids de bases d'oxydation par rapport au poids total de la composition.

11. Composition tinctoriale selon l'une des revendications 7 à 10, **caractérisée en ce qu'**elle comprend en outre un coupleur choisi parmi les méta-phénylènediamines, les méta-aminophénols, les méta-diphénols, les coupleurs naphtaléniques, les coupleurs hétérocycliques et leurs sels d'addition.

12. Composition selon la revendication 11, **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

13. Composition selon la revendication 11 ou 12, **caractérisée en ce que** le ou les coupleurs sont présents en une quantité comprise entre 0,001 et 20 %, de préférence entre 0,005 et 6 % en poids par rapport au poids total de la composition.

14. Composition selon l'une des revendications 7 à 13, **caractérisée en ce qu'**elle comprend un ou plusieurs colorants directs additionnels différents des composés de formule (I), choisis parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs tétraazapentaméthiniques, les colorants directs indoaminiques et les colorants directs naturels.

15. Composition selon la revendication 16, **caractérisé en ce** la ou les colorants directs additionnels sont présents en une quantité comprise entre 0,001 à 20 % en poids et de préférence entre 0,005 et 10% en poids par rapport au poids total de la composition.

16. Composition selon l'une quelconque des revendications 7 à 15, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

17. Composition selon l'une quelconque des revendications 7 à 16, **caractérisé en ce qu'**elle comprend au moins un solvant hydroxylé, tel que l'éthanol, le propylène glycol, le glycérol, les mono éthers de polyols.

18. Composition selon l'une quelconque des revendications 7 à 17, **caractérisé en ce qu'**elle comprend au moins un adjuvant choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents conditionneurs tels que les silicones volatiles ou non volatiles, modifiées ou non modifiées, les agents filmogènes, les céramides, les agents conservateurs, les agents opacifiants.

19. Procédé de teinture des fibres kératiniques, **caractérisé en ce qu'**il comprend les étapes suivantes :
- on applique la composition tinctoriale selon l'une des revendications 7 à 18 sur les fibres kératiniques,
- on laisser pauser pendant une période comprise entre 3 minutes et 1 heure et de préférence entre 15 minutes et 45 minutes, puis
- on rince lesdites fibres.

20. Utilisation d'une composition selon l'une des revendications 7 à 18 pour la teinture des fibres kératiniques.

21. Dispositif à plusieurs compartiments ou « Kit » de coloration, **caractérisé par le fait qu'**il comporte un premier compartiment renfermant une composition telle que définie selon l'une des revendications 7 à 18 et un second compartiment renfermant une composition comprenant un agent oxydant.

22. Composé cationique azoïque à motif julolidine de formule (I) : 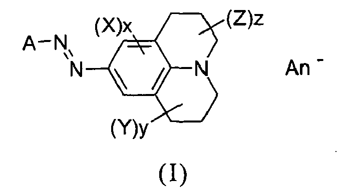 dans laquelle
A représente un hétérocycle aromatique cationique choisi parmi les composés de formules générales suivantes : 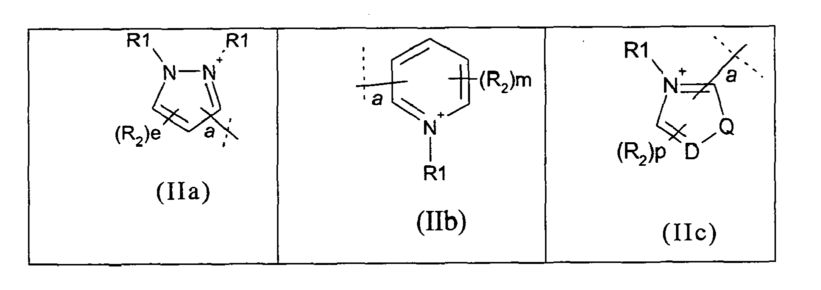 dans lesquelles :
R₁ représentent indépendamment les uns des autres :
• une chaîne hydrocarbonée en C₁-C₁₆, linéaire ou ramifiée, saturée ou insaturée, pouvant former un ou plusieurs cycles carbonés comportant de 3 à 7 chaînons, éventuellement condensés avec le cycle aromatique, éventuellement substituée, éventuellement interrompue par un ou plusieurs groupements choisis parmi les hétéroatomes tels que l'oxygène, l'azote ou le soufre, et le groupement carbonyle; R₁ ne comportant pas de liaison nitro, nitroso, peroxyde et diazo ; R₁ étant directement rattaché à l'atome d'azote, quaternisé ou non, du cycle hétéroaromatique A par l'intermédiaire d'un atome de carbone.
R₂ représente indépendamment les uns des autres :
• une chaîne hydrocarbonée en C₁-C₁₆, linéaire ou ramifiée, saturée ou insaturée, pouvant former un ou plusieurs cycles carbonés aromatique ou non, comportant de 3 à 6 chaînons, éventuellement substituée, éventuellement interrompue par un ou plusieurs hétéroatomes ou par un ou plusieurs groupements portant au moins un hétéroatome, de préférence choisis parmi l'oxygène, l'azote ;
• un groupement hydroxyle,
• un groupement alkyl-oxy en C₁-C₄,
• un groupement (poly)hydroxyalkyloxy en C₂-C₄ ;
• un groupement alkyloxycarbonyle (R₁₁O-CO-) dans lequel R₁₁ représente un radical alkyle en C₁-C₄,
• un radical alkylcarbonyloxy (R₁₂CO-O-) dans lequel R₁₂ représente un radical alkyle est en C₁-C₄;
• un groupement amino, un groupement amino substitué par un ou deux radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote, par exemple l'oxygène, le soufre ;
• un groupement alkylcarbonylamino (R₁₃CO-NR₁₃-) et/ou (R₁₃CO-NH-) dans lequel les radicaux R₁₃ indépendamment l'un de l'autre, représentent un radical alkyle en C₁-C₄;
• un groupement carbamoyle ((R₁₄)₂N-CO) dans lequel les radicaux R₁₄ indépendamment l'un de l'autre , identiques ou non, représentent un atome d'hydrogène, un radical alkyle en C₁-C₄;
• un groupement uréido ((R₁₅)₂N-CO-NR₁₆-) dans lequel les radicaux R₁₅ et R₁₆, indépendamment l'un de l'autre, représentent un atome d'hydrogène, un radical alkyle en C₁-C₄;
• un groupement sulfonamide ((R₁₇)₂N-SO₂-) dans lequel les radicaux R₁₇, indépendamment l'un de l'autre, représentent un atome d'hydrogène, un radical alkyle en C₁-C₄, ;
• un groupement alkylsulfonylamino (R₁₈SO₂-NR₁₉-) dans lequel les radicaux R₁₈, R₁₉, indépendamment l'un de l'autre, représentent un atome d'hydrogène, un radical alkyle en C₁-C₄;
• un groupement guanidinium ((R₂₀)₂N-C(=NH₂+)-NR₂₁-) dans lequel les radicaux R₂₀ et R₂₁, indépendamment les uns des autres, représentent un atome d'hydrogène, un radical alkyle en C₁-C₄;
• un groupement nitro ;
• un groupement cyano ;
• un atome d'halogène, de préférence le chlore, le fluor ;
deux radicaux R₂, portés par des atomes de carbone adjacents peuvent former ensemble avec l'atome de carbone auquel chacun est rattaché, un cycle condensé, aromatique ou non ;
m représente un entier compris entre 0 et 4 ;
e est un entier compris entre 0 et 2 ;
p est un entier entre 0 et 1 ;
D représente un groupe CR₂ ou un atome d'azote ;
Q représente un groupe NR₁, ou un atome d'oxygène ou de soufre ;
la liaison a issue des formules (IIa), (IIb) ou (IIc), relie le groupement A au groupement azoïque ;
dans le cas des formules (IIa), (IIb) ou (IIc) et lorsque deux radicaux R₂ portés par deux atomes de carbone adjacents forment un cycle aromatique, la liaison a peut relier le groupement A au groupement azoïque par l'intermédiaire dudit cycle aromatique ;
l'électroneutralité des composés étant assurée par un ou plusieurs anions An⁻, identiques ou non, cosmétiquement acceptables ;
X, Y et Z sont définis comme possibilités de substitutions respectivement sur les cycles alkyles et aryle du noyau tricyclique ;
x est compris entre 0 et 2, y est compris entre 0 et 6, et z est compris entre 0 et 6, x, y et z étant entiers ;
ou leurs sels d'addition ou leurs solvates ;
lorsqu'un radical alkyle ou la partie alkyle d'un radical est dit(e) 'substitué(e)', alors il ou elle comprend au moins un substituant choisi parmi les groupements :
• hydroxyle,
• alkyloxy en C₁-C₄, (poly)hydroxyalkyloxy en C₂-C₄,
• amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote ;
lorsqu'un radical aryle ou hétéroaryle ou la partie aryle ou hétéroaryle d'un radical est dit(e) 'substitué(e)', comme par exemple le substituant X sur le noyau aromatique du tricycle, alors il ou elle comprend au moins un substituant porté par un atome de carbone, choisi parmi :
• un radical alkyle en C₁-C₁₆, de préférence en C₁-C₈, éventuellement substitué par un ou plusieurs radicaux choisis parmi les radicaux hydroxy, alkyloxy en C₁-C₂, (poly)-hydroxyalkyloxy en C₂-C₄, acylamino, amino substitué par un ou deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
• un atome d'halogène tel que chlore, fluor ou brome ;
• un groupement hydroxyle ;
• un radical alkyloxy en C₁-C₂ ;
• un radical (poly)-hydroxyalkyloxy en C₂-C₄ ;
• un radical amino ;
• un radical amino substitué par un ou deux radicaux alkyles, identiques ou différents, en C₁-C₄ éventuellement porteurs d'au moins un groupement hydroxyle;
• un radical acylamino (-N₃₁R-COR₃₂) dans lequel le radical R₃₁ est un atome d'hydrogène, un radical alkyle en C₁-C₄ éventuellement porteur d'au moins un groupement hydroxyle et le radical R₃₂ est un radical alkyle en C₁-C₂ ;
• un radical carbamoyle ((R₃₃)₂N-CO-) dans lequel les radicaux R₃₃, identiques ou non, représentent un atome d'hydrogène, un radical alkyle en C₁-C₄ éventuellement porteur d'au moins un groupement hydroxyle ;
• un radical alkylsulfonylamino (R₃₄SO₂-N₃₅R-) dans lequel le radical R₃₄ représente un atome d'hydrogène, un radical alkyle en C₁-C₄ éventuellement porteur d'au moins un groupement hydroxyle et le radical R₃₅ représente un radical alkyle en C₁-C₄, un radical phényle ;
• un radical aminosulfonyle ((R₃₆)₂N-SO₂-) dans lequel les radicaux R₃₆, identiques ou non, représentent un atome d'hydrogène, un radical alkyle en C₁-C₄ éventuellement porteur d'au moins un groupement hydroxyle.
lorsque la partie cyclique ou hétérocyclique d'un radical non aromatique est dit(e) 'substitué(e)', comme par exemple les substituants Y et Z des hétérocycles aliphatiques du tricycle, alors il ou elle comprend au moins un substituant porté par un atome de carbone choisi parmi les groupements :
• hydroxyle,
• alkyloxy en C₁-C₄, (poly)hydroxyalkyloxy en C₂-C₄,
• alkylcarbonylamino ((R₄₁CO-NR₄₂-) dans lequel le radical R₄₂ est un atome d'hydrogène, un radical alkyle en C₁-C₄ éventuellement porteur d'au moins un groupement hydroxyle et le radical R₄₁ est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote ;
lorsqu'un cycle ne porte pas le nombre maximum de substituants, alors la ou les positions non susbtituées portent un atome d'hydrogène ;
à l'exception de
2-(9-julolidylazo)-3-méthylbenzothiazolium perchlorate
2-(9-julolidylazo)-3-éthylthiazolium perchlorate.

23. Composé selon la revendication 22, **caractérisé en ce que** R₁ représente un groupement alkyle ou hydroxyalkyle en C₁-C₈.

24. Composé selon la revendication 22 ou 23, **caractérisé en ce que** les formules (IIa), (IIb) et (IIc) sont telles qu'elles comportent deux radicaux R₂ portés par des atomes de carbone adjacents, ces dits radicaux formant alors ensemble avec l'atome de carbone auquel chacun est rattaché, un cycle condensé aromatique éventuellement substitué.

25. Composé selon l'une quelconque des revendications 22 à 24, **caractérisé en ce que** e, m et p valent 0.

26. Composé selon l'une quelconque des revendications 22 à 25, **caractérisé en ce** x vaut 0 ou x vaut 1 avec X représentant un groupe alkyle, hydroxyle, hydroxyalkyle, alkyloxy, amino, alkylamino, dialkylamino, acylamino.

27. Composé selon l'une quelconque des revendications 22 à 26, **caractérisé en ce que** le composé de formule (I) possède l'une des structures suivantes :
